# EUROPEAN PATENT APPLICATION

(11) **EP 2 799 419 A1**
(43) Date of publication of application: **05.11.2014**
(21) Application number: 12862925.0
(22) Date of filing: 21.12.2012
(51) Int. Cl.: C07C 45/34, C07C 47/21, B01J 31/22, C07B 61/00

(54) **METHOD FOR PRODUCING FARNESAL USING VANADIUM COMPLEX**

(30) Priority: 26.12.2011 JP 2011284112
(71) Applicant: Sagami Chemical Research Institute, Ayase-shi Kanagawa 252-1193 (JP); Manac Inc., Fukuyama-shi, Hiroshima 721-0956 (JP)
(72) Inventor: INOUE, Munenori, Ayase-shi Kanagawa 252-1193 (JP); ARAKI, Hiroshi, Ayase-shi Kanagawa 252-1193 (JP); TANAKA, Takeshi, Fukuyama-shi Hiroshima 721-0956 (JP)
(74) Representative: Hoffmann Eitle
(86) International application number: PCT/JP2012/083343
(87) International publication number: WO 2013/099819

(57) **Abstract**

The present invention provides a method for producing farnesal that is useful as a production intermediate of pharmaceuticals, agricultural chemicals and perfumes. More specifically, the present invention provides a method for producing farnesal (3), comprising reacting (E)-nerolidol (1) with an oxidizing agent in the presence of a vanadium complex of the general formula (2): 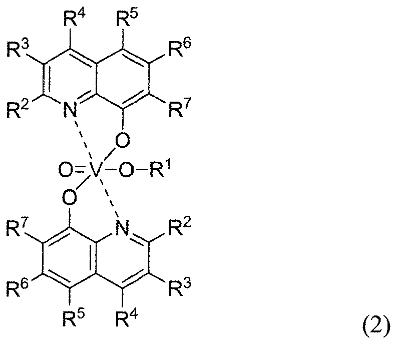 wherein R¹ to R⁷ are the same as defined in the description and claims.

## Description

### TECHNICAL FIELD

The present invention relates to a method for producing farnesal from (E)-nerolidol using a vanadium complex. In addition, the present invention relates to a novel vanadium complex and a production method thereof.

### BACKGROUND ART

Farnesal (3,7,11-trimethyl-2,6,10-dodecatrienal) is known to be an important compound used as a production intermediate of pharmaceuticals, agricultural chemicals, perfumes and the like. (2E,6E)-farnesal in particular can be used as a production intermediate of polyisoprenoid derivatives that are useful as anticancer agents and the like (see, for example, Patent Document 1). Since farnesal has four types of isomers consisting of a (2E,6E) form, (2Z,6E) form, (2E,6Z) form and (2Z,6Z) form, various studies have been conducted on a method for selectively producing (2E,6E)-farnesal.

For example, methods have been disclosed for obtaining (2E,6E)-farnesal by using (2E,6E)-farnesol ((2E,6E)-3,7,11-trimethyl-2,6,10-dodecatrien-1-ol) as raw material (see, for example, Patent Document 1 and Non-Patent Document 1). In all of these methods, it is necessary to use (2E,6E)-farnesol as raw material. However, farnesol also has four types of isomers in the same manner as farnesal, and a method has yet to be established for efficiently and selectively obtaining the (2E,6E) form thereof.

In addition, methods are also known for obtaining farnesal from the inexpensive nerolidol (3,7,11-trimethyl-1,6,10-dodecatrien-3-ol). For example, methods have been disclosed for synthesizing an isomer mixture of (2E,6E)-farnesal and (2Z,6E)-farnesal from (E)-nerolidol in a single step using a chromic acid oxidizing agent (see, for example, Non-Patent Documents 2 and 3). However, in the case of carrying out the reaction using a chromic acid oxidizing agent, the ratio of (2E,6E)-farnesal in the resulting isomer mixture is low, and since a large amount of tar components are formed after the reaction, ordinary post-treatment becomes difficult. Moreover, since this method uses an excess of a highly toxic chromium compound, it cannot be applied to industrial production, and particularly the production of pharmaceuticals.

Moreover, an example of another method for obtaining farnesal from nerolidol consists of synthesizing in a single step using tetrahydrolinalyl orthovanadate as catalyst

(see, for example, Patent Document 2). However, in addition to there being no description regarding the E/Z isomeric ratio of the raw material or product, this method is also difficult to carry out industrially in terms of catalyst availability.

On the other hand, a vanadium complex has been reported that can be prepared from a vanadium compound having an oxidation number of IV or V, 8-quinolinol and a lower alcohol for use as a catalyst used in the oxidation of alcohols to aldehydes (see, for example, Non-Patent Document 4). However, examples have not been reported of its use in a reaction for oxidizing to an unsaturated aldehyde accompanying isomerization of a tertiary allylic alcohol in the manner of (E)-nerolidol.

### PRIOR ART DOCUMENTS

### PATENT DOCUMENTS

Patent Document 1: Japanese Unexamined Patent Publication No. 2003-40826
Patent Document 2: U.S. Patent No. 3944623

### NON-PATENT DOCUMENTS

Non-Patent Document 1: Journal of American Chemical Society, 124(14), 3647-3655, 2002
Non-Patent Document 2: Synthesis, (5), 356-364, 1979
Non-Patent Document 3: Chemistry - A European Journal, 15(44), 11918-11927,2009
Non-Patent Document 4: Organic Letters, 13(8), 1908-1911,2011

### DISCLOSURE OF THE INVENTION

### PROBLEMS TO BE SOLVED BY THE INVENTION

Conventional methods for producing (2E,6E)-farnesal using (E)-nerolidol have the shortcomings of being unable to be carried out industrially, having poor efficiency, or the resulting (2E,6E)-farnesal being excessively costly. An object of the present invention is to provide a method for efficiently and inexpensively producing farnesal having a high ratio of the (2E,6E) isomer from (E)-nerolidol.

### MEANS FOR SOLVING THE PROBLEMS

As a result of conducting extensive studies in consideration of the aforementioned problems, the inventors of the present invention found that farnesal having a high ratio of (2E,6E) isomer can be easily produced by reacting (E)-nerolidol with an oxidizing agent in the presence of a vanadium complex, and discovered a novel vanadium complex, along with a production method thereof, that can be used in that production of farnesal, thereby leading to completion of the present invention.

Namely, the present invention relates to a method for producing farnesal of the following formula (3): 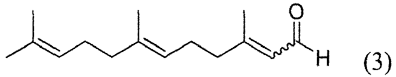 comprising: reacting (E)-nerolidol of the following formula (1): 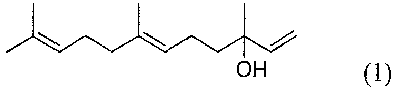 with an oxidizing agent in the presence of a vanadium complex of the following general formula (2): 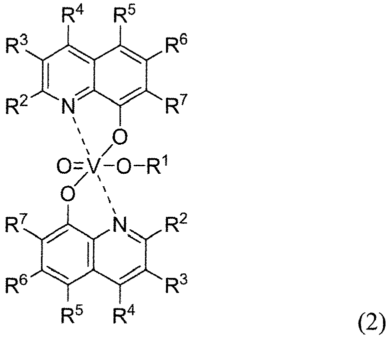 (wherein, R¹ represents an alkyl group having 1 to 20 carbon atoms, a cycloalkyl group having 3 to 8 carbon atoms, an alkenyl group having 2 to 20 carbon atoms, an aryl group having 6 to 18 carbon atoms, an aralkyl group having 7 to 20 carbon atoms, or an acyl group having 2 to 7 carbon atoms, and
R², R³, R⁴, R⁵, R⁶ and R⁷ each independently represent a hydrogen atom, alkyl group having 1 to 6 carbon atoms, haloalkyl group having 1 to 6 carbon atoms, alkoxy group having 1 to 6 carbon atoms, alkylthio group having 1 to 6 carbon atoms, phenyl group, nitro group, cyano group, carboxyl group or halogen atom).

In addition, the present invention relates to a method for producing farnesal of the following formula (3): 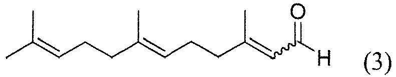 comprising: reacting (E)-nerolidol of the following formula (1): 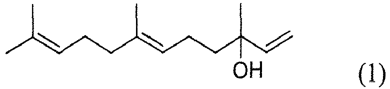 with an oxidizing agent in the presence of a vanadium compound selected from a compound of the following general formula (4a): 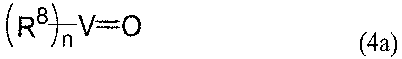 (wherein, n represents 2 or 3, and R⁸ represents an R'O- group or either alone or together represents an alkanedionate having 2 to 8 carbon atoms, where R' represents an alkyl group having 1 to 6 carbon atoms or a cycloalkyl group having 3 to 6 carbon atoms) or a metavanadate salt, a compound of the following general formula (5):

R¹OH (5)

(wherein, R¹ is the same as defined above), and an 8-quinolinol derivative of the following general formula (6): 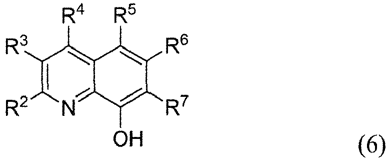 (wherein, R², R³, R⁴, R⁵, R⁶ and R⁷ are the same as defined above).

In addition, the present invention relates to a vanadium complex of the following general formula (2a): 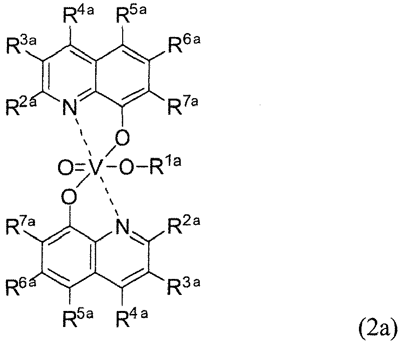 (wherein, R^{1a} represents an alkyl group having 1 to 20 carbon atoms, an alkenyl group having 2 to 20 carbon atoms, an aryl group having 6 to 18 carbon atoms or an aralkyl group having 7 to 20 carbon atoms,
R^{2a}, R^{3a}, R^{4a}, R^{5a}, R^{6a} and R^{7a} each independently represent a hydrogen atom, alkyl group having 1 to 6 carbon atoms, haloalkyl group having 1 to 6 carbon atoms, alkoxy group having 1 to 6 carbon atoms, phenyl group, nitro group, cyano group, carboxyl group or halogen atom,
provided that, in the case R^{2a}, R^{3a}, R^{4a}, R^{5a}, R^{6a} and R^{7a} are all hydrogen atoms, R^{1a} is not an alkyl group having 1 to 12 carbon atoms, an allyl group or a benzyl group, in the case R^{2a} is a methyl group and R^{3a}, R^{4a}, R^{5a}, R^{6a} and R^{7a} are hydrogen atoms, R^{1a} is not an alkyl group having 1 to 4 carbon atoms, in the case R^{2a}, R^{3a}, R^{4a}, R^{6a} and R^{7a} are hydrogen atoms and R^{5a} is a methyl group or nitro group, R^{1a} is not an ethyl group, and in the case R^{2a}, R^{3a}, R^{4a}, R^{6a} and R^{7a} are hydrogen atoms and R^{5a} is a halogen atom or R^{2a}, R^{3a}, R^{4a} and R^{6a} are hydrogen atoms and R^{5a} and R^{7a} are halogen atoms, R^{1a} is not an alkyl group having 1 to 5 carbon atoms).

Moreover, the present invention relates to a method for producing a vanadium complex of the following general formula (2a): 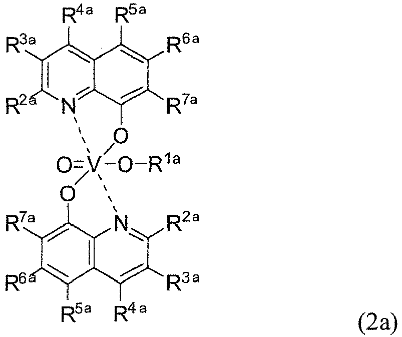 (wherein, R^{1a}, R^{2a}, R^{3a}, R^{4a}, R^{5a}, R^{6a} and R^{7a} are the same as defined above), comprising: reacting a vanadium compound selected from a compound of the following general formula (4a): 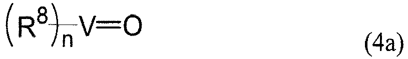 (wherein, n and R⁸ are the same as defined above) or a metavanadate salt with an 8-quinolinol derivative of the following general formula (6a): 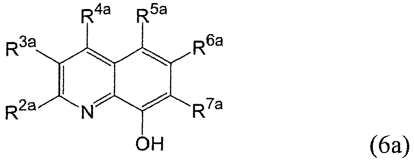 (wherein, R^{2a}, R^{3a}, R^{4a}, R^{5a}, R^{6a} and R^{7a} are the same as defined above), and an oxidizing agent as necessary, in the presence (in the case R^{1a} ≠ R') or in the absence (in the case R^{1a} = R') of an alcohol of the following general formula (5a):

R^{1a}OH (5a)

(wherein, R^{1a} is the same as defined above).

### EFFECTS OF THE INVENTION

The present invention is effective as a method for efficiently, and without using highly toxic reagents, producing farnesal, and particularly (2E,6E)-farnesal, which is useful as a production intermediate of pharmaceuticals, agricultural chemicals and perfumes, from inexpensive raw materials.

### BEST MODE FOR CARRYING OUT THE INVENTION

The following provides a detailed explanation of embodiments of the present invention.

### [Definition of Terms]

First, an explanation is provided of terms used in the present description and scope of claim for patent. Each term has the meaning indicated below unless specifically indicated otherwise.

The term "alkyl group having 1 to 20 carbon atoms" refers to a monovalent group of a linear or branched, saturated aliphatic hydrocarbon having 1 to 20 carbon atoms, and examples thereof include a methyl group, ethyl group, propyl group, isopropyl group, butyl group, isobutyl group, sec-butyl group, tert-butyl group, pentyl group, hexyl group, 2-ethylhexyl group, octyl group, nonyl group, decyl group, dodecyl group and octadecyl group. Similarly, an "alkyl group having 1 to 6 carbon atoms" refers to a monovalent group of a linear or branched, saturated aliphatic hydrocarbon having 1 to 6 carbon atoms, and examples thereof include a methyl group, ethyl group, propyl group, isopropyl group, butyl group, isobutyl group, sec-butyl group, tert-butyl group, pentyl group and hexyl group.

The term "cycloalkyl group having 3 to 8 carbon atoms" refers to a monovalent group of a cyclic, saturated aliphatic hydrocarbon having 3 to 8 carbon atoms, and examples thereof include a cyclopropyl group, cyclobutyl group, cyclopentyl group, cyclohexyl group and cyclooctyl group. Similarly, a "cycloalkyl group having 3 to 6 carbon atoms" refers to a monovalent group of a cyclic, saturated aliphatic hydrocarbon having 3 to 6 carbon atoms, and examples thereof include a cyclopropyl group, cyclobutyl group, cyclopentyl group and cyclohexyl group. Furthermore, the terms "cycloalkyl group having 3 to 8 carbon atoms" and "cycloalkyl group having 3 to 6 carbon atoms" include aspects in which the aforementioned monovalent group of a cyclic, saturated aliphatic hydrocarbon is substituted with an alkyl group having 1 to 6 carbon atoms or a hydroxyl group and the like. Examples thereof include a 2-methylcyclopropyl group, 1-methylcyclopentyl group, 3-hydroxycyclopentyl group, 4-methylcyclohexyl group and 2-hydroxycyclohexyl group.

The term "alkenyl group having 2 to 20 carbon atoms" refers to a monovalent group of a linear or branched, unsaturated aliphatic hydrocarbon having 2 to 20 carbon atoms, and examples thereof include an ethenyl group, allyl group, 2-butenyl group, 3-butenyl group, 3-methyl-2-butenyl group, 3-hexenyl group, oleyl group, (E)-3,7-dimethyl-2,6-octadienyl group, 3,7,11-trimethyl-1,6,10-dodecatrien-3-yl group and 3,7,11-trimethyl-2,6,10-dodecatrienyl group. Similarly, unless specifically indicated otherwise, an "alkenyl group having 2 to 6 carbon atoms" refers to a monovalent group of a linear or branched, unsaturated aliphatic hydrocarbon having 2 to 6 carbon atoms, and examples thereof include an ethenyl group, allyl group, 2-butenyl group, 3-butenyl group, 3-methyl-2-butenyl group and 3-hexenyl group.

The term "aryl group having 6 to 18 carbon atoms" refers to a monovalent group of an aromatic compound having 6 to 18 carbon atoms, and examples thereof include a phenyl group, naphthyl group, anthryl group and pyridyl group. Furthermore, the term "aryl group having 6 to 18 carbon atoms" includes aspects in which the aforementioned monovalent group of an aromatic compound is substituted with an alkyl group having 1 to 6 carbon atoms, an alkoxy group having 1 to 6 carbon atoms or a halogen atom and the like. Examples thereof include a 2-methylphenyl group, 3-methylphenyl group, 4-methylphenyl group, 4-methyl-2,6-di-t-butyl group, 4-methoxyphenyl group, 2-chlorophenyl group and 4-chlorophenyl group.

The term "aralkyl group having 7 to 20 carbon atoms" refers to an arylalkyl group having 7 to 20 carbon atoms (here, the aryl moiety is an aryl group having 6 to 18 carbon atoms and the alkyl moiety is an alkyl group having 1 to 6 carbon atoms), and examples thereof include a benzyl group, 4-methoxybenzyl group, phenethyl group and pyridylmethyl group.

The term "acyl group having 2 to 7 carbon atoms" refers to an RCO- group (here, R represents an alkyl group having 1 to 6 carbon atoms, an alkenyl group having 2 to 6 carbon atoms, a haloalkyl group having 1 to 6 carbon atoms or a phenyl group), and examples thereof include an acetyl group, propionyl group, butyryl group, isobutyryl group, valeryl group, isovaleryl group, pivaloyl group, acryloyl group, methacryloyl group, crotonoyl group, benzoyl group and trifluoroacetyl group.

The term "haloalkyl group having 1 to 6 carbon atoms" refers to an alkyl group having 1 to 6 carbon atoms substituted with one or more halogen atoms, and examples thereof include a bromomethyl group, 2-bromoethyl group, 3-bromopropyl group, 4-bromobutyl group, 5-bromopentyl group, 6-bromohexyl group, iodomethyl group, 2-iodoethyl group, 3-iodopropyl group, 4-iodobutyl group, 5-iodopentyl group, 6-iodohexyl group, fluoromethyl group, 2-fluoroethyl group, 3-fluoropropyl group, 4-fluorobutyl group, 5-fluoropentyl group, 6-fluorohexyl group, tribromomethyl group, trichloromethyl group and trifluoromethyl group.

The term "alkoxy group having 1 to 6 carbon atoms" refers to an R'O- group (here, R' represents an alkyl group having 1 to 6 carbon atoms or a cycloalkyl group having 3 to 6 carbon atoms), and examples thereof include a methoxy group, ethoxy group, propyloxy group, isopropyloxy group, cyclopropyloxy group, butoxy group, isobutyloxy group, sec-butyloxy group, tert-butyloxy group, hexyloxy group, cyclobutyloxy group, cyclopentyloxy group and cyclohexyloxy group.

The term "alkylthio group having 1 to 6 carbon atoms" refers to an R"S group (here, R" represents an alkyl group having 1 to 6 carbon atoms or a cycloalkyl group having 3 to 6 carbon atoms), and examples thereof include a methylthio group, ethylthio group, propylthio group, isopropylthio group, cyclopropylthio group, butylthio group, isobutylthio group, sec-butylthio group, tert-butylthio group, hexylthio group, cyclobutylthio group, cyclopentylthio group and cyclohexylthio group.

The terms "halogen atom" and "halo" are interchangeable, and refer to an iodine atom, bromine atom, chlorine atom or fluorine atom.

### [Production Method of Farnesal (3)]

The following provides a detailed description of a method for producing farnesal (3) of the present invention.

The method for producing farnesal (3) of the present invention is characterized by reacting (E)-nerolidol (1) with an oxidizing agent in the presence of a vanadium complex (2). The starting material, (E)-nerolidol (1) is available commercially, and can be easily acquired from a reagent supplier such as Sigma-Aldrich Corp. In addition, it can be synthesized in compliance with a known method (for example, the method described in Japanese Unexamined Patent Publication No. H2-4726).

The vanadium complex (2) used in the method for producing the farnesal (3) of the present invention is represented by the following general formula (2): 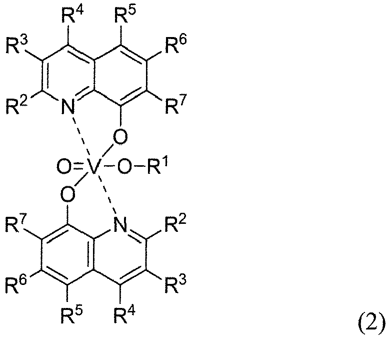 (wherein, R¹, R², R³, R⁴, R⁵, R⁶ and R⁷ are the same as defined above).

In the complex of the general formula (2), that in which R¹ is an alkyl group having 1 to 20 carbon atoms is preferable from the viewpoint of favorable yield. In addition, that in which R¹ is an alkenyl group having 2 to 20 carbon atoms, and in particular a 3,7,11-trimethyl-1,6,10-dodecatrien-3-yl group (namely, that derived from nerolidol) or a 3,7,11-trimethyl-2,6,10-dodecatrienyl group (namely, that derived from farnesol) is preferable from the viewpoint of ease of preparation. Moreover, that in which R², R³, R⁴, R⁵, R⁶ and R⁷ in the complex of the general formula (2) each independently represent a hydrogen atom or halogen atom is preferable from the viewpoints of favorable yield and ease of preparation. In particular, that in which R², R³, R⁴, R⁵, R⁶ and R⁷ are hydrogen atoms, that in which R², R³, R⁴ and R⁶ are hydrogen atoms and R⁵ and R⁷ are chlorine atoms, that in which R², R³, R⁴ and R⁶ are hydrogen atoms and R⁵ and R⁷ are fluorine atoms, and that in which R², R³, R⁴ and R⁶ are hydrogen atoms and R⁵ and R⁷ are bromine atoms are preferable.

Although the amount of the vanadium complex (2) used is a so-called catalytic amount, from the viewpoint of reaction efficiency, it is preferably 0.05 moles to 0.2 moles, and more preferably 0.05 moles to 0.1 mole, based on 1 mole of (E)-nerolidol (1).

There are no particular limitations on the oxidizing agent used in the method for producing the farnesal (3) of the present invention, and examples of oxidizing agents that can be used include air, oxygen and sulfoxides such as dimethylsulfoxide, dibutylsulfoxide, diphenylsulfoxide or tetramethylenesulfoxide. Two or more types of these oxidizing agents may also be used as a mixture. From the viewpoints of productivity and handling ease, air, oxygen, dimethylsulfoxide, tetramethylenesulfoxide or a mixture of these oxidizing agents is used preferably.

In the case of using oxygen as an oxidizing agent in the production method of the present invention, the oxygen can be used as a mixture with another gas, and for example, the oxygen can be used by mixing with air or an inert gas (such as nitrogen or helium).

In the case of using air or oxygen as an oxidizing agent in the production method of the present invention, there are no particular limitations on the method used to supply the air or oxygen, and for example, a method consisting of replacing a liquid phase contacted by the reaction solution with air or oxygen, a method consisting of passing air or oxygen through a liquid phase contacted by the reaction solution, or a method consisting of blowing air or oxygen into the reaction solution, can be used.

In the case of using a sulfoxide such as dimethylsulfoxide, dibutylsulfoxide, diphenylsulfoxide or tetramethylenesulfoxide for the oxidizing agent in the production method of the present invention, the amount used is preferably 1 mole to 20 moles, more preferably 1 mole to 10 moles and even more preferably 1 mole to 5 moles based on 1 mole of the E-nerolidol (1) from the viewpoints of reaction velocity and reaction efficiency.

The method for producing the farnesal (3) of the present invention may be carried out in a solvent, there are no particular limitations on solvents able to be used provided they are solvents that are inert in the reaction, and can be suitably selected corresponding to the desired reaction temperature. Examples of solvents that can be used include halogenated aromatic hydrocarbon-based solvents such as chlorobenzene, bromobenzene, dichlorobenzene or trichlorobenzene, halogenated aliphatic hydrocarbon-based solvents such as dichloromethane, dibromomethane, chloroform, carbon tetrachloride, ethylene dichloride, 1,1,1-trichloroethane, trichloroethylene or pentachloroethane, aromatic hydrocarbon-based solvents such as benzene, toluene, xylene or mesitylene, aliphatic hydrocarbon-based solvents such as hexane, heptane, octane or cyclohexane, and aromatic nitrile-based solvents such as benzonitrile, and two or more types of these solvents may be used by mixing. A halogenated aromatic hydrocarbon-based solvent, aromatic hydrocarbon-based solvent or aromatic nitrile-based solvent is used preferably, a halogenated aromatic hydrocarbon-based solvent is used particularly preferably from the viewpoint of yield, and chlorobenzene, dichlorobenzene, trichlorobenzene or a mixed solvent thereof is used more preferably. The amount of solvent used is preferably 3 times to 50 times (weight basis), and more preferably 4 times to 30 times (weight basis), the amount of the E-nerolidol (1).

The method for producing the farnesal (3) of the present invention can be carried out at a temperature suitably selected from a range from room temperature to 180°C. The method is preferably carried out at 80°C to 140°C, and more preferably at 110°C to 130°C, from the viewpoint of favorable yield.

The farnesal (3) can be isolated and purified from a solution following the reaction as necessary. There are no particular limitations on the isolation/purification method, and the farnesal (3) can be isolated and purified by a method known among persons with ordinary skill in the art, such as solvent extraction, distillation, silica gel chromatography, fractional thin layer chromatography, fractional liquid chromatography or other commonly used methods.

### [Production of Vanadium Complex (2)]

The vanadium complex (2) used in the method for producing the farnesal (3) of the present invention may be prepared by reacting a vanadium compound (4) with an 8-quinolinol derivative (6) in the presence or absence of an alcohol/acid (5). This reaction may be further carried out in the presence of an oxidizing agent as necessary.

The vanadium compound (4) is selected from a compound of the following general formula (4a): 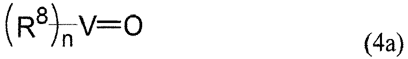 (wherein, n and R⁸ are the same as defined above) or a metavanadate salt (4b).

In the case n is 2 (namely, vanadium (IV)), the compound of the general formula (4a) is preferably that in which R⁸ either alone or together represents an alkanedionate having 2 to 8 carbon atoms such as ethanedionate (oxalate), pentane-2,4-dionate (acetylacetonate) or octane-2,4-dionate. In the case n is 3 (namely, vanadium (V)), that in which R⁸ represents an R'O- group (here, R' represents an alkyl group having 1 to 6 carbon atoms or a cycloalkyl group having 3 to 6 carbon atoms), namely an alkoxy group having 1 to 6 carbon atoms, is preferable, while that in which R⁸ represents an alkoxy group having 1 to 4 carbon atoms is more preferable. The metavanadate salt (4b) is preferably an alkaline metal salt (such as a sodium salt or potassium salt) or an ammonium salt of metavanadic acid. For example, triethoxyvanadium (V) oxide, triisopropoxyvanadium (V) oxide, bis(acetylacetonato)oxovanadium (IV), vanadium (IV) oxyoxalate, sodium metavanadate or ammonium metavanadate are available commercially, and can be easily acquired from a reagent supplier such as Sigma-Aldrich Corp. Triisopropoxyvanadium (V) oxide, bis(acetylacetonato)oxovanadium (IV) and ammonium metavanadate are preferable from the viewpoints of handling and ease of acquisition.

The alcohol/acid (5) is an alcohol or carboxylic acid compound of the following general formula (5):

R¹OH (5)

(wherein, the definition of R¹ and preferable aspects thereof are the same as defined above). Thus, preferable examples of the alcohol/acid (5) include saturated alcohols having 1 to 20 carbon atoms, unsaturated alcohols having 2 to 20 carbon atoms, and particularly 3,7,11-trimethyl-1,6,10-dodecatrien-3-ol (namely nerolidol) or 3,7,11-trimethyl-2,6,10-dodecatrien-1-ol (namely, farnesol), and saturated carboxylic acids having 2 to 7 carbon atoms, and particularly acetic acid. These are available commercially, and can be easily acquired from a reagent supplier such as Sigma-Aldrich Corp. The amount of the alcohol/acid (5) used is 1 mole or more based on 1 mole of the vanadium compound (4). However, when the vanadium compound (4), especially a compound of the general formula (4a) in which R⁸ represents an R'O-group, is reacted with the 8-quinolinol derivative (6) and an oxidizing agent as necessary in the absence of the alcohol/acid (5), a vanadium complex of the general formula (2) in which R¹ = R' can be obtained. Thus, in the case R¹ = R', production of the vanadium complex (2) can be carried out in the absence of the alcohol/acid (5).

The 8-quinolinol derivative (6) is represented by the following general formula (6): 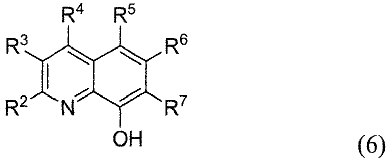 (wherein, the definitions of R², R³, R⁴, R⁵, R⁶ and R⁷ and preferable aspects thereof are the same as defined above). Thus, preferable examples of the 8-quinolinol derivative (6) include 8-quinolinol, 5-fluoro-8-quinolinol, 5,7-difluoro-8-quinolinol, 5,7-dichloro-8-quinolinol and 5,7-dibromo-8-quinolinol. A portion of these are available commercially, and can be acquired from a reagent supplier such as Sigma-Aldrich Corp. In addition, the 8-quinolinol derivative can be easily prepared in accordance with known methods. The amount of the 8-quinolinol derivative used is 2 moles or more based on 1 mole of the vanadium compound (4).

An oxidizing agent can be used as necessary to produce the vanadium complex (2). There are no particular limitations on oxidizing agents able to be used, and examples of oxidizing agents that can be used include air, oxygen and sulfoxides such as dimethylsulfoxide, dibutylsulfoxide, diphenylsulfoxide and tetramethylenesulfoxide. Two or more types of these oxidizing agents may be mixed. From the viewpoints of productivity and handling ease, air, oxygen, dimethylsulfoxide, tetramethylenesulfoxide or a mixture thereof is used preferably. The usage method and amount used of these oxidizing agents are in compliance with that previously described [Production Method of Farnesal (3)].

Production of the vanadium complex (2) may be carried out in a solvent, there are no particular limitations on solvents able to be used provided they are solvents that are inert in the reaction, and can be suitably selected corresponding to the desired reaction temperature. Examples of solvents that can be used include halogenated aromatic hydrocarbon-based solvents such as chlorobenzene, bromobenzene, dichlorobenzene or trichlorobenzene, halogenated aliphatic hydrocarbon-based solvents such as dichloromethane, dibromomethane, chloroform, carbon tetrachloride, ethylene dichloride, 1,1,1-trichloroethane, trichloroethylene orpentachloroethane, aromatic hydrocarbon-based solvents such as benzene, toluene, xylene or mesitylene, aliphatic hydrocarbon-based solvents such as hexane, heptane, octane or cyclohexane, and nitrile-based solvents such as acetonitrile, propionitrile or benzonitrile. The alcohol/acid (5) may also be used in excess as a solvent. Two or more types of these solvents may be used by mixing. From the viewpoint of yield, a halogenated aromatic hydrocarbon-based solvent, halogenated aliphatic hydrocarbon-based solvent, nitrile-based solvent or mixed solvent thereof is used more preferably.

Production of the vanadium complex (2) can be carried out at a temperature suitably selected from a range from 0°C to 180°C. Room temperature is preferable from the viewpoint of favorable yield.

The vanadium complex (2) can be isolated and purified from a solution following the reaction as necessary. There are no particular limitations on the isolation/purification method, and the vanadium complex (2) can be isolated and purified by a method known among persons with ordinary skill in the art, such as solvent extraction, recrystallization, silica gel chromatography, fractional thin layer chromatography, fractional liquid chromatography or other commonly used methods.

### [Production method using vanadium complex (2) formed in reaction system]

The vanadium complex (2) used in the method for producing the farnesal (3) of the present invention may also be that formed in the reaction system. For example, as a result of having prepared the vanadium complex (2) in accordance with a method like that previously described, the resulting vanadium complex (2) may also be used in the subsequent reaction with the (E)-nerolidol (1) without isolating or purifying. Alternatively, the method for producing the farnesal (3) of the present invention can also be carried out by reacting the (E)-nerolidol (1) with an oxidizing agent in the presence of the vanadium compound (4), the alcohol/acid (5) and the 8-quinolinol derivative (6).

Thus, the present invention also provides a method for producing the farnesal (3) that is characterized by reacting the (E)-nerolidol (1) with an oxidizing agent in the presence of the vanadium compound (4), the alcohol/acid (5) and the 8-quinolinol derivative (6). In this production method, the amount of the vanadium compound (4) is preferably 0.05 moles to 0.2 moles, and more preferably 0.05 moles to 0.1 mole, based on 1 mole of the (E)-nerolidol (1). The amounts of the alcohol/acid (5) and the 8-quinolinol derivative (6) with respect to the vanadium compound (4) are as defined above. In this production method, the alcohol/acid (5) may not be added separately, as the (E)-nerolidol (1) plays the role of the alcohol.

### [Novel vanadium complex (2a) and production method thereof]

The present invention provides a novel vanadium complex of the following general formula (2a): 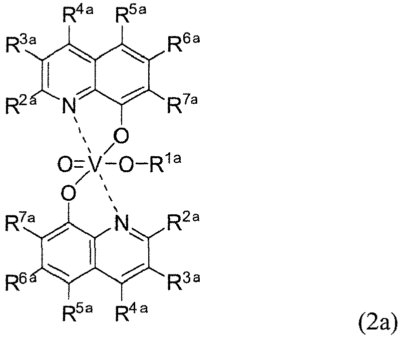 (wherein, R^{1a}, R^{2a}, R^{3a}, R^{4a}, R^{5a}, R^{6a} and R^{7a} are the same as defined above).

This novel vanadium complex can be produced by reacting the vanadium compound (4) in the presence or absence of an alcohol of the following general (5a):

R^{1a}OH (5a)

(wherein R^{1a} is the same as defined above) with an 8-quinolinol derivative of the following general formula (6a): 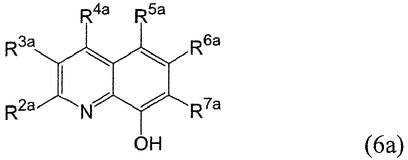 (wherein, R^{2a}, R^{3a}, R^{4a}, R^{5a}, R^{6a} and R^{7a} are the same as defined above) and an oxidizing agent as necessary. The reaction conditions thereof comply with that described in the aforementioned [Production of Vanadium Complex (2)]. Similarly, in the case R^{1a} = R', production of the vanadium complex (2a) can be carried out in the absence of the alcohol (5a).

### EXAMPLES

Although the following indicates examples for clarifying embodiments of the present invention, the present invention is not limited to only the following examples.

Reaction solutions obtained in the examples were analyzed by gas chromatography followed by calculating the area percentages of the purities of (2E,6E)-farnesal and (2Z,6E)-farnesal. The measurement conditions were as indicated below.
Apparatus: GC-14A (Shimadzu Corp.)
Column: HP-ULTRA1 (Agilent Technologies Inc.), 25 m x I.D. 0.32 mm, 0.52 µmdf
Column temperature: 100°C → (10°C/min) → 250°C
Injection temperature: 250°C
Carrier gas: Helium gas
Detector: Hydrogen flame ionization detector (FID)

### In addition, measurement conditions used when determining the NMR spectra of compounds isolated in the reference examples and examples were as indicated below.

Solutions were prepared by mixing the compounds with chloroform-d (CDCl₃, Cambridge Isotope Laboratories, Inc., containing 0.05% TMS) followed by ¹H-NMR measurement with a nuclear magnetic resonance device (NMR, Avance 400, Bruker Corp.).

### Reference Example 1

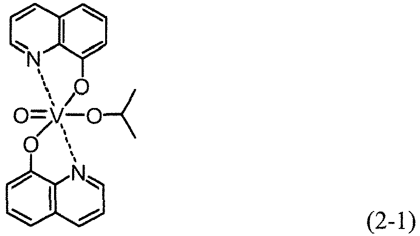

Bis(acetylacetonato)oxovanadium (IV) (3.64 g, 20.6 mmol) was suspended in 2-propanol (100 mL) followed by the addition of 8-quinolinol (5.97 g, 41.1 mmol). After stirring this for 7 hours at room temperature, the precipitated crystals were filtered and washed with 2-propanol (20 mL). The resulting wet crystals were dried for 17 hours at 40°C to obtain a vanadium complex of the aforementioned formula (2-1) (to be referred to as "i-Pr complex", 6.42 g, yield: 75.2%).

¹H-NMR(400MHz, CDCl₃) δ8.60(s, 1H), 8.46(s, 1H), 8.09(d, 1H, J=8.0Hz), 8.02(d, 1H, J=8.4Hz), 7.51-7.56(m, 2H), 7.15-7.22(m, 6H), 6.26-6.32(m, 1H), 1.53(d, 3H, J=11.6Hz), 1.49(d, 3H, J=6.4Hz)

### Production Example 1

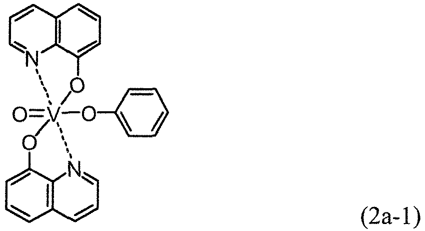

### Synthesis of Vanadium Complex of Formula (2a-1)

Bis(acetylacetonato)oxovanadium (IV) (182 mg, 0.69 mmol) was suspended in acetonitrile (5 mL) followed by the addition of 8-quinolinol (199 mg, 1.37 mmol) and phenol (5 mL). After stirring this for 6 hours at room temperature, the solvent was distilled off under reduced pressure. After adding diisopropyl ether (10 mL) to the residue, the precipitated crystals were filtered and washed with diisopropyl ether (5 mL). The resulting wet crystals were dried for 7 hours at 40°C to obtain a vanadium complex of the aforementioned formula (2a-1) (264 mg, yield: 85.7%).

¹H-NMR(400MHz, CDCl₃) δ8.64(d, 1H, J=4.4Hz), 8.49(d, 1H, J=3.2Hz), 8.15(d, 1H, J=7.2Hz), 8.04(d, 1H, J=7.2Hz), 7.54-7.60(m, 2H), 7.27-7.31 (m, 6H), 7.17-7.23(m, 4H), 6.95-6.99(m, 1H)

### Production Example 2

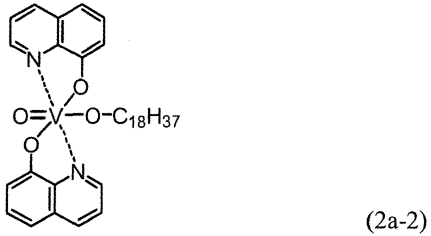

### Synthesis of Vanadium Complex of Formula (2a-2)

Bis(acetylacetonato)oxovanadium (IV) (182 mg, 0.69 mmol) was suspended in acetonitrile (5 mL) followed by the addition of 8-quinolinol (199 mg, 1.37 mmol) and 1-octadecanol (370 mg, 1.37 mmol). After stirring this for 5 hours at room temperature, the precipitated crystals were filtered and washed with acetonitrile (5 mL). After suspending the resulting wet crystals in tetrahydrofuran (5 mL) and stirring for 1 hour at room temperature, the crystals were filtered and washed with tetrahydrofuran (5 mL). The resulting wet crystals were dried for 16 hours at 40°C to obtain a vanadium complex of the aforementioned formula (2a-2) (285 mg, yield: 66.4%).

¹H-NMR(400MHz, CDCl₃) δ(ppm): 8.61(d, 1H, J=4.8Hz), 8.47(d, 1H, J=4.4Hz), 8.11(d, 1H, J=8.3Hz), 8.03(d, 1H, J=8.2Hz), 7.52(t, 2H, J=7.8Hz), 7.14-7.25(m, 6H), 6.03(dt, 1H, J=6.5, 11.1Hz), 5.63(dt, 1H, J=6.5, 11.1Hz), 1.85(t, 2H, 6.5Hz), 1.16-1.43(m, 30H), 0.88(t, 3H, J=7.0Hz)

### Production Example 3

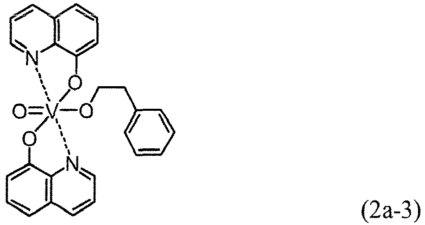

### Synthesis of Vanadium Complex of Formula (2a-3)

8-Quinolinol (618 mg, 4.3 mmol) was dissolved in acetonitrile (7 mL) followed by the addition of triisopropoxyvanadium (V) oxide (0.5 mL, 2.1 mmol) and phenethyl alcohol (3.8 mL, 32 mmol) and stirring for 18 hours at room temperature. After distilling off the solvent of the reaction solution under reduced pressure, adding hexane to the resulting residue to solidify, and filtering the solid that formed, the resulting solid was dried under reduced pressure to obtain the vanadium complex of the aforementioned formula (2a-3) (445 mg, yield: 44%).

¹H-NMR(400MHz, CDCl₃) δ(ppm): 8.61(d, 1H, J=4.6Hz), 8.44(d, 1H, J=4.6Hz), 8.12(d, 1H, J=8.2Hz), 8.04(d, 1H, J=8.2Hz), 7.56(dd, 1H, J=4.6, 8.2Hz), 7.54(dd, 1H, J=4.6, 8.2Hz), 7.12-7.30(m, 7H), 7.06-7.09(4H, m), 6.12(ddd, 1H, J=6.5, 8.0, 14.5Hz), 5.80(ddd, 1H, J=6.5, 8.0, 14.5Hz), 3.27(ddd, 1H, J=6.5, 8.0, 13.8Hz), 3.11(ddd, 1H, J=6.5, 8.0, 13.8Hz)

### Production Example 4

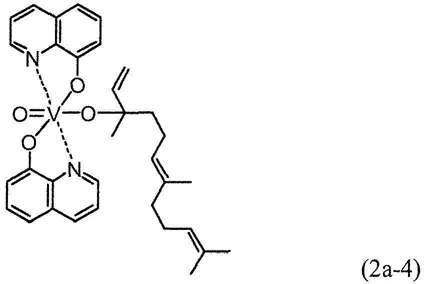

### Synthesis of Vanadium Complex of Formula (2a-4)

Bis(acetylacetonato)oxovanadium (IV) (182 mg, 0.69 mmol) was suspended in acetonitrile (5 mL) followed by the addition of 8-quinolinol (199 mg, 1.37 mmol) and (E)-nerolidol (305 mg, 1.37 mmol). After stirring this for 5 hours at room temperature, diisopropyl ether (5 mL) was injected. The precipitated crystals were filtered and washed with diisopropyl ether (5 mL). The resulting wet crystals were dried for 6 hours at 40°C under reduced pressure to obtain a vanadium complex of the aforementioned formula (2a-4) (232 mg, yield: 58.6%).

### Production Example 5

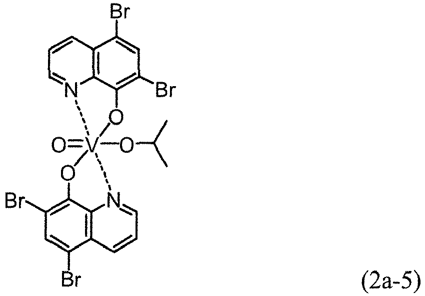

### Synthesis of Vanadium Complex of Formula (2a-5)

5,7-Dibromo-8-quinolinol (1.29 g, 4.3 mmol) was dissolved in acetonitrile (7 mL) followed by the addition of triisopropoxyvanadium (V) oxide (0.5 mL, 2.1 mmol) and stirring for 24 hours at room temperature. The precipitate that formed was filtered and the resulting solid was washed with diethyl ether followed by drying under reduced pressure to obtain a vanadium complex of the aforementioned formula (2a-5) (977 mg, yield: 63%).

¹H-NMR(400MHz, CDCl₃) δ(ppm): 8.60(dd, 1H, J=1.2, 4.8Hz), 8.40(dd, 1H, J=1.3, 4.5Hz), 8.37(dd, 1H, J=1.3, 8.4Hz), 8.31(dd, 1H, J=1.2, 8.6Hz), 8.00(s, 1H), 7.93(s, 1H), 7.44(dd, 1H, J=4.8, 8.6Hz), 7.38(dd, 1H, J=4.5, 8.4Hz), 6.54(sept, 1H, J=6.1Hz), 1.62(d, 3H, J=6.1Hz), 1.46(d, 3H, J=6.1Hz)

### Production Example 6

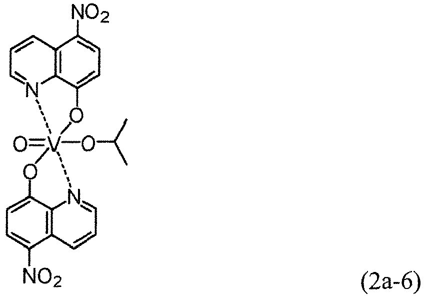

### Synthesis of Vanadium Complex of Formula (2a-6)

5-Nitro-8-quinolinol (810 mg, 4.3 mmol) was dissolved in acetonitrile (7 mL) followed by the addition of triisopropoxyvanadium (V) oxide (0.5 mL, 2.1 mmol) and stirring for 24 hours at room temperature. The precipitate that formed was filtered and the resulting solid was washed with diethyl ether followed by drying under reduced pressure to obtain a vanadium complex of the aforementioned formula (2a-6) (974 mg, yield: 91%).

¹H-NMR(400MHz, CDCl₃) δ(ppm): 9.45(dd, 1H, J=1.2, 8.8Hz), 9.32(dd, 1H, J=1.3, 8.8Hz), 8.77(d, 1H, J=8.8Hz), 8.72(d, 1H, J=8.8Hz), 8.71(dd, 1H, J=1.2, 4.5Hz), 8.50(dd, 1H, J=1.3, 4.5Hz), 7.61(dd, 1H, J=4.5, 8.8Hz), 7.53(dd, 1H, J=4.5, 8.8Hz), 7.20(d, 1H, J=4.5Hz), 7.14(d, 1H, J=4.5Hz), 6.56(sept, 1H, J=6.2Hz), 1.57(d, 3H, J=6.2Hz), 1.50(d, 3H, J=6.2Hz)

### Production Example 7

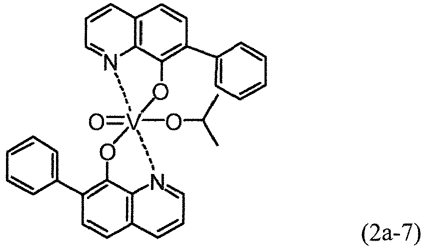

### Synthesis of Vanadium Complex of Formula (2a-7)

7-Phenyl-8-quinolinol (111 mg, 0.50 mmol) was dissolved in acetonitrile (1 mL) followed by the addition of triisopropoxyvanadium (V) oxide (59 µL, 0.25 mmol) and stirring for 24 hours at room temperature. The precipitate that formed was filtered and the resulting solid was washed with diethyl ether followed by drying under reduced pressure to obtain a vanadium complex of the aforementioned formula (2a-7) (71 mg, yield: 50%).

¹H-NMR(400MHz, CDCl₃) δ(ppm): 8.44(dd, 1H, J=1.3, 4.5Hz), 8.34(dd, 1H, J=1.3, 4.5Hz), 8.23(dd, 2H, J=1.3, 8.5Hz), 8.17(dd, 2H, J=1.3, 8.5Hz), 8.05(dd, 1H, J=1.3, 8.2Hz), 8.00(dd, 1H, J=1.3, 8.2Hz), 7.86(d, 1H, J=8.5Hz), 7.80(d, 1H, J=8.5Hz), 7.59(m, 2H), 7.54(m, 2H), 7.42(m, 1H), 7.39(m, 1H), 7.29(d, 1H, J=8.5Hz), 7.22(d, 1H, J=8.5Hz), 7.15(dd, 1H, J=4.5, 8.2Hz), 7.12(dd, 1H, J=4.5, 8.2Hz), 6.31 (sept, 1H, J=6.1Hz), 1.55(d, 3H, J=6.1Hz), 1.47(d, 3H, J=6.1Hz)

### Production Example 8

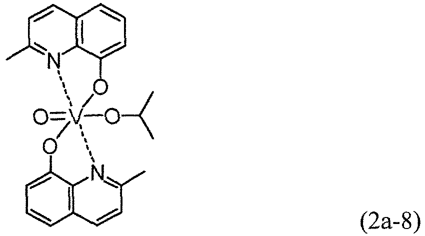

### Synthesis of Vanadium Complex of Formula (2a-8)

2-Methyl-8-quinolinol (678 mg, 4.3 mmol) was dissolved in acetonitrile (7 mL) followed by the addition of triisopropoxyvanadium (V) oxide (0.5 mL, 2.1 mmol) and stirring for 24 hours at room temperature. The precipitate that formed was filtered and the resulting solid was washed with diethyl ether followed by drying under reduced pressure to obtain a vanadium complex of the aforementioned formula (2a-8) (817 mg, yield: 87%).

¹H-NMR(400MHz, CDCl₃) δ(ppm): 7.84(d, 2H, J=8.3Hz), 7.38(t, 2H, J=7.8Hz), 7.07(d, 2H, J=8.3Hz), 7.00(d, 2H, J=7.8Hz), 6.99(d, 2H, J=7.8Hz), 6.41(sept, 1H, J=6.1Hz), 2.93(s, 6H), 1.52(d, 6H, J=6.1Hz)

### Production Example 9

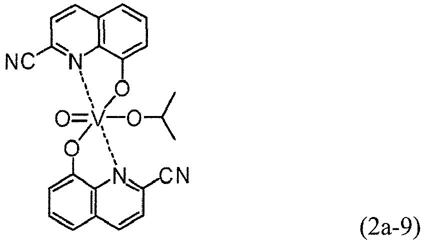

### Synthesis of Vanadium Complex of Formula (2a-9)

2-Cyano-8-quinolinol (289 mg, 1.7 mmol) was dissolved in acetonitrile (3 mL) followed by the addition of triisopropoxyvanadium (V) oxide (0.2 mL, 0.85 mmol) and stirring for 24 hours at room temperature. The precipitate that formed was filtered and the resulting solid was washed with diethyl ether followed by drying under reduced pressure to obtain a vanadium complex of the aforementioned formula (2a-9) (382 mg, yield: 97%).

¹H-NMR(400MHz, CDCl₃) δ(ppm): 8.25(d, 2H, J=8.3Hz), 7.74(t, 2H, J=8.0Hz), 7.58(d, 2H, J=8.3Hz), 7.41(d, 2H, J=8.0Hz), 7.29(d, 2H, J=8.0Hz), 6.43(sept, 1H, J=6.2Hz), 1.52(d, 6H, J=6.2Hz)

### Production Example 10

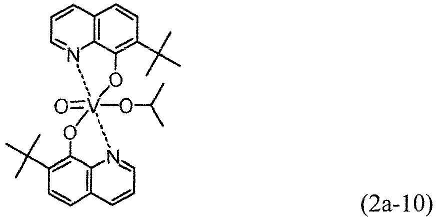

### Synthesis of Vanadium Complex of Formula (2a-10)

7-tert-Butyl-8-quinolinol (341 mg, 1.7 mmol) was dissolved in acetonitrile (3 mL) followed by the addition of triisopropoxyvanadium (V) oxide (0.2 mL, 0.85 mmol) and stirring for 16 hours at room temperature. The precipitate that formed was filtered and the resulting solid was washed with diethyl ether followed by drying under reduced pressure to obtain a vanadium complex of the aforementioned formula (2a-10) (322 mg, yield: 72%).

¹H-NMR(400MHz, CDCl₃) δ(ppm): 8.49(dd, 1H, J=1.4, 4.5Hz), 8.35(dd, 1H, J=1.4, 4.5Hz), 8.02(dd, 1H, J=1.4, 8.3Hz), 7.95(dd, 1H, J=1.4, 8.3Hz), 7.64(d, 1H, J=8.6Hz), 7.63(d, 1H, J=8.6Hz), 7.16(d, 1H, J=8.6Hz), 7.10(dd, 1H, J=4.5, 8.3Hz), 7.09(d, 1H, J=8.6Hz), 7.06(dd, 1H, J=4.5, 8.3Hz), 6.23(sept, 1H, J=6.1Hz), 1.73(s, 9H), 1.69(s, 9H), 1.53(d, 3H, J=6.1Hz), 1.43(d, 3H, J=6.1Hz)

### Production Example 11

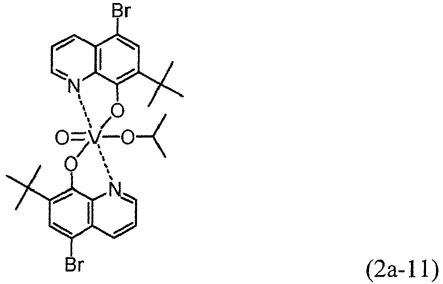

### Synthesis of Vanadium Complex of Formula (2a-11)

5-Bromo-7-tert-butyl-8-quinolinol (479 mg, 1.7 mmol) was dissolved in methylene chloride (5 mL) followed by the addition of triisopropoxyvanadium (V) oxide (0.2 mL, 0.85 mmol) and stirring for 23 hours at room temperature. The solvent in the reaction solution was distilled off under reduced pressure followed by drying the resulting solid under reduced pressure to obtain a vanadium complex of the aforementioned formula (2a-11) (554 mg, yield: 97%).

### Production Example 12

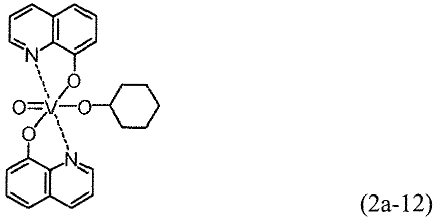

### Synthesis of Vanadium Complex of Formula (2a-12)

Bis(acetylacetonato)oxovanadium (IV) (182 mg, 0.69 mmol) was suspended in acetonitrile (5 mL) followed by the addition of 8-quinolinol (199 mg, 1.37 mmol) and cyclohexanol (5 mL). After stirring this for 20 hours at room temperature, the solvent was distilled off under reduced pressure. After adding diisopropyl ether (10 mL) to the residue, the precipitate that formed was filtered and the resulting solid was washed with diisopropyl ether followed by drying under reduced pressure to obtain a vanadium complex of the aforementioned formula (2a-12) (266 mg, yield: 85%).

¹H-NMR(400MHz, CDCl₃) δ(ppm): 8.61(d, 1H, J=3.9Hz), 8.46(d, 1H, J=3.9Hz), 8.10(d, 1H, J=8.4Hz), 8.03(d, 1H, J=8.4Hz), 7.54(m, 2H), 7.14-7.31(m, 6H), 6.04(m, 1H), 2.30(m, 2H), 1.29-1.78(m, 7H), 1.14(m,lH)

### Examples 1 to 15

(E)-Nerolidol (100 mg, 0.45 mmol) was dissolved in chlorobenzene (MCB, 2.0 g) or o-dichlorobenzene (DCB, 2.0 g) followed by the addition of the vanadium complexes indicated in Table 1 (0.045 mmol) and reacting at 130°C in the presence of flowing air. After cooling to room temperature, the formation of (2E,6E)-farnesal and (2Z,6E)-farnesal was confirmed by gas chromatography analysis. The GC yields and isomeric ratios ((2E,6E)-farnesal/(2Z,6E-farnesal) are shown in Table 1.

**[Table 1]**

| | Example 1 | Example 2 | Example 3 | Example 4 | Example 5 |
|---|---|---|---|---|---|
| Vanadium complex | 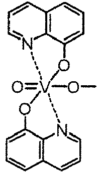 | 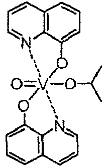 | 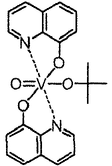 | 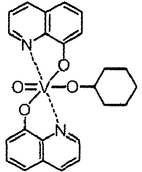 | 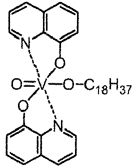 |
| Reaction time | 22 | 11 | 8 | 10 | 7 |
| Solvent | MCB | MCB | MCB | MCB | MCB |
| 2E, 6E% | 59.2 | 68.0 | 70.0 | 68.0 | 73.8 |
| 2Z, 6E% | 4.3 | 8.1 | 12.1 | 6.5 | 10.3 |
| Isomeric ratio | 13.77 | 8.40 | 5.79 | 10.46 | 7.17 |

| | Example 6 | Example 7 | Example 8 | Example 9 | Example 10 |
|---|---|---|---|---|---|
| Vanadium complex | 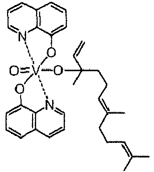 | 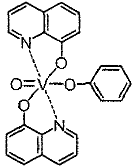 | 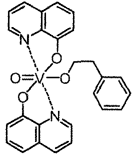 | 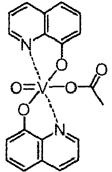 | 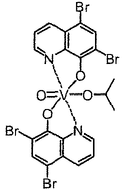 |
| Reaction time | 23 | 11 | 9 | 9 | 10 |
| Solvent | MCB | MCB | MCB | MCB | DCB |
| 2E, 6E% | 75.8 | 61.7 | 71.0 | 57.7 | 66.5 |
| 2Z, 6E% | 5.5 | 8.1 | 6.1 | 9.4 | 5.7 |
| Isomeric ratio | 13.8 | 7.62 | 11.6 | 6.14 | 11.67 |

| | Example 11 | Example 12 | Example 13 | Example 14 | Example 15 |
|---|---|---|---|---|---|
| Vanadium complex | 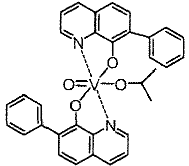 | 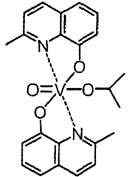 | 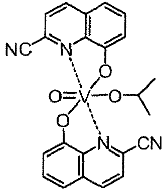 | 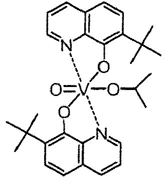 | 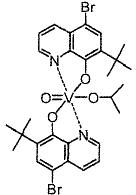 |
| Reaction time | 10 | 24 | 9 | 15 | 8 |
| Solvent | MCB | MCB | MCB | MCB | MCB |
| 2E, 6E% | 66.6 | 45.9 | 37.7 | 56.0 | 52.7 |
| 2Z, 6E% | 10.0 | 6.3 | 11.0 | 3.4 | 10.1 |
| Isomeric ratio | 6.66 | 7.29 | 3.43 | 16.47 | 5.22 |

| | | | | | |
|---|---|---|---|---|---|
| MCB: monochlorobenzene DCB: o-dichlorobenzene | | | | | |

### Example 16

(E)-Nerolidol (100 mg, 0.45 mmol) was dissolved in o-dichlorobenzene (2.0 g) followed by the addition of i-Pr complex (18.6 mg, 0.045 mmol) and reacting at 130°C in the presence of flowing air. After cooling to room temperature, the formation of (2E,6E)-farnesal and (2Z,6E)-farnesal was confirmed by gas chromatography analysis. The GC yields and isomeric ratio ((2E,6E)-farnesal/(2Z,6E)-farnesal) are shown in Table 2. The solvent of the reaction solution was distilled off under reduced pressure and the resulting residue was purified by flash column chromatography (hexane:ethyl acetate = 20:1, Rf value = 0.5) to obtain an isomeric mixture of (2E,6E)-farnesal and (2Z,6E)-farnesal (47 mg, yield: 47.5%, (2E,6E)-farnesal:(2Z,6E)-farnesal = 81.2:11.4, isomeric ratio: (2E,6E)-farnesal/(2Z,6E)-farnesal = 7.12).

(2E,6E)-farnesal: ¹H-NMR(400MHz, CDCl₃) δ9.99(d, 1H, J=8.0Hz), 5.89(d, 1H, J=8.0Hz), 5.03-5.13(m, 2H), 2.18-2.29(m, 4H), 2.17(d, 3H, J=1.2Hz), 2.02-2.11(m, 2H), 1.94-2.02(m, 2H), 1.68(s, 3H), 1.61(s, 3H), 1.60(s, 3H);
(2Z,6E)-farnesal: ¹H-NMR(400MHz, CDCl₃) δ9.92(d, 1H, J=8.2Hz), 5.88(d, 1H, J=8.2Hz), 5.03-5.16(m, 2H), 2.60(t, 2H, J=7.5Hz), 2.21-2.30(m, 2H), 2.01-2.10(m, 2H), 1.94-2.01(m, 2H), 1.99(d, 3H, J=1.2Hz), 1.68(s, 3H), 1.60(s, 6H)

### Examples 17 to 19

(E)- Nerolidol (100 mg, 0.45 mmol) was dissolved in the solvents (2.0 g) shown in Table 2 followed by the addition of i-Pr complex (18.6 mg, 0.045 mmol) and reacting at 130°C in the presence of flowing air. After cooling to room temperature, the formation of (2E,6E)-farnesal and (2Z,6E)-farnesal was confirmed by gas chromatography analysis. The GC yields and isomeric ratios ((2E,6E)-farnesal/(2Z,6E-farnesal) are shown in Table 2.

**[Table 2]**

| | Example 16 | Example 17 | Example 18 | Example 19 |
|---|---|---|---|---|
| Solvent | o-dichlorobenzene | 1,3,5-trichlorobenzene | o-xylene | Benzonitrile |
| Reaction time | 5 | 5 | 6 | 6 |
| 2E, 6E% | 70.0 | 66.5 | 66.6 | 51.1 |
| 2Z, 6E% | 8.3 | 7.4 | 9.4 | 6.9 |
| Isomeric ratio | 8.43 | 8.99 | 7.09 | 7.41 |

### Example 20

(E)- Nerolidol (100 mg, 0.45 mmol) was dissolved in chlorobenzene (2.0 g) followed by the addition of i-Pr complex (9.3 mg, 0.0225 mmol) and reacting for 23 hours at 130°C in the presence of flowing air. After cooling to room temperature, (2E,6E)-farnesal (GC content: 57.1%) and (2Z,6E)-farnesal (GC content: 5.8%) were confirmed to have been obtained (isomeric ratio: (2E,6E)-farnesal/(2Z,6E)-farnesal = 9.84).

### Example 21

(E)- Nerolidol (100 mg, 0.45 mmol) was dissolved in chlorobenzene (2.0 g) followed by the addition of i-Pr complex (18.6 mg, 0.045 mmol) and reacting for 23 hours at 100°C in the presence of flowing air. After cooling to room temperature, (2E,6E)-farnesal (GC content: 70.1%) and (2Z,6E)-farnesal (GC content: 8.4%) were confirmed to have been obtained (isomeric ratio: (2E,6E)-farnesal/(2Z,6E)-farnesal = 8.35).

### Example 22

(E)- Nerolidol (100 mg, 0.45 mmol) was dissolved in chlorobenzene (2.0 g) followed by the addition of i-Pr complex (18.6 mg, 0.045 mmol) and dimethylsulfoxide (70 mg, 0.9 mmol) and reacting for 21 hours at 130°C. After cooling to room temperature, (2E,6E)-farnesal (GC content: 66.4%) and (2Z,6E)-farnesal (GC content: 8.6%) were confirmed to have been obtained (isomeric ratio: (2E,6E)-farnesal/(2Z,6E)-farnesal = 7.72).

### Examples 23 to 25

(E)- Nerolidol (100 mg, 0.45 mmol) was dissolved in chlorobenzene (2.0 g) followed by the addition of 8-quinolinol (13 mg, 0.09 mmol) and the vanadium compounds (0.045 mmol) indicated in Table 3 and reacting at 130°C in the presence of flowing air. After cooling to room temperature, the formation of (2E,6E)-farnesal and (2Z,6E)-farnesal was confirmed by gas chromatography analysis. The GC contents and isomeric ratios ((2E,6E)-farnesal/(2Z,6E)-farnesal) are shown in Table 3.

**[Table 3]**

| | Example 23 | Example 24 | Example 25 |
|---|---|---|---|
| Vanadium compound | VO(acac)₂ | VO(OiPr)₃ | NH₄VO₃ |
| Reaction time | 21 | 7 | 22 |
| 2E, 6E% | 69.9 | 68.5 | 81.1 |
| 2Z, 6E% | 6.6 | 5.5 | 10.0 |
| Isomeric ratio | 10.56 | 12.45 | 8.11 |

| | | | |
|---|---|---|---|
| VO(acac)₂: Bis(acetylacetonato)oxovanadium (IV) VO(OiPr)₃: Triisopropoxyvanadium (V) oxide NH₄VO₃: Ammonium metavanadate | | | |

### Example 26

(E)- Nerolidol (1 g, 4.5 mmol) was dissolved in chlorobenzene (5 g) followed by the addition of ammonium metavanadate (53 mg, 0.45 mmol) and 8-quinolinol (131 mg, 0.90 mmol) and reacting for 12 hours at 130°C while bubbling air through the reaction solution. After cooling to room temperature, the chlorobenzene and vanadium complex were removed from the resulting reaction solution using flash column chromatography (hexane:ethyl acetate = 10:1) to obtain a crude product in the form of a yellow oil (589 mg, (2E,6E)-farnesal (GC content: 65.4%) and (2Z,6E)-farnesal (GC content: 8.04%) (isomeric ratio: (2E,6E)-farnesal/(2Z,6E)-farnesal = 8.1). This crude product was then purified by flash column chromatography (hexane:ethyl acetate = 20:1, Rf value = 0.5) to obtain an isomeric mixture of (2E,6E)-farnesal and (2Z,6E)-farnesal (478 mg, yield: 48%, (2E,6E)-farnesal:(2Z,6E)-farnesal = 68.7:10.9 (isomeric ratio: (2E,6E)-farnesal/(2Z,6E)-farnesal = 6.3).

### Example 27

(E)- Nerolidol (100 mg, 0.45 mmol) was dissolved in chlorobenzene (2 g) followed by the addition of ammonium metavanadate (5.3 mg, 0.045 mmol) and 8-quinolinol (13 mg, 0.09 mmol) and reacting for 12 hours at 130°C in an oxygen atmosphere. After cooling to room temperature, (2E,6E)-farnesal (GC content: 67.7%) and (2Z,6E)-farnesal (GC content: 7.6%) were confirmed to have been obtained (isomeric ratio: (2E,6E)-farnesal/(2Z,6E)-farnesal = 8.91).

### Example 28

(E)- Nerolidol (100 mg, 0.45 mmol) was dissolved in chlorobenzene (2 g) followed by the addition of ammonium metavanadate (5.3 mg, 0.045 mmol), 5,7-dibromo-8-quinolinol (27 mg, 0.09 mmol) and dimethylsulfoxide (70 mg, 0.9 mmol) and reacting for 10 hours at 130°C. After cooling to room temperature, (2E,6E)-farnesal (GC content: 81.6%) and (2Z,6E)-farnesal (GC content: 18.4%) were confirmed to have been obtained (isomeric ratio: (2E,6E)-farnesal/(2Z,6E)-farnesal = 4.43).

### Example 29

(E)- Nerolidol (100 mg, 0.45 mmol) was dissolved in chlorobenzene (2 g) followed by the addition of ammonium metavanadate (5.3 mg, 0.045 mmol), 5,7-dichloro-8-quinolinol (19 mg, 0.09 mmol) and dimethylsulfoxide (70 mg, 0.9 mmol) and reacting for 7.5 hours at 130°C. After cooling to room temperature, (2E,6E)-farnesal (GC content: 86.8%) and (2Z,6E)-farnesal (GC content: 13.2%) were confirmed to have been obtained (isomeric ratio: (2E,6E)-farnesal/(2Z,6E)-farnesal = 6.58).

### Example 30

(E)- Nerolidol (100 mg, 0.45 mmol) was dissolved in chlorobenzene (2 g) followed by the addition of ammonium metavanadate (5.3 mg, 0.045 mmol), 5-fluoro-8-quinolinol (15 mg, 0.09 mmol) and dimethylsulfoxide (70 mg, 0.9 mmol) and reacting for 8.5 hours at 130°C. After cooling to room temperature, (2E,6E)-farnesal (GC content: 92.3%) and (2Z,6E)-farnesal (GC content: 7.7%) were confirmed to have been obtained (isomeric ratio: (2E,6E)-farnesal/(2Z,6E)-farnesal = 11.99).

### Example 31

(E)- Nerolidol (1 g, 4.5 mmol) was dissolved in chlorobenzene (5 g) followed by the addition of ammonium metavanadate (53 mg, 0.45 mmol), 8-quinolinol (131 mg, 0.90 mmol) and dimethylsulfoxide (704 mg, 9.0 mmol) and reacting for 7 hours at 130°C while bubbling air through the reaction solution. After cooling to room temperature, the chlorobenzene and vanadium complex were removed from the resulting reaction solution using flash column chromatography (hexane:ethyl acetate = 10:1) to obtain a crude product in the form of a yellow oil (633 mg, (2E,6E)-farnesal (GC content: 62.6%) and (2Z,6E)-farnesal (GC content: 8.8%) (isomeric ratio: (2E,6E)-farnesal/(2Z,6E)-farnesal = 7.1). This crude product was then purified by flash column chromatography (hexane:ethyl acetate = 20:1, Rf value = 0.5) to obtain an isomeric mixture of (2E,6E)-farnesal and (2Z,6E)-farnesal (457 mg, yield: 46%, (2E,6E)-farnesal:(2Z,6E)-farnesal = 66.5:11.0 (isomeric ratio: (2E,6E)-farnesal/(2Z,6E)-farnesal = 6.0).

### INDUSTRIAL APPLICABILITY

According to the production method of the present invention, farnesal, and particularly (2E,6E)-farnesal, which is useful as a production intermediate of pharmaceuticals, agricultural chemicals and perfumes, can be efficiently produced from inexpensive raw materials without using highly toxic reagents. Accordingly, the method of the present invention can be used as a production method on an industrial scale.

## Claims

1. A method for producing farnesal of the following formula (3): 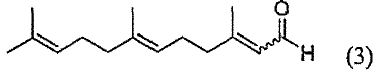 comprising: reacting (E)-nerolidol of the following formula (1): 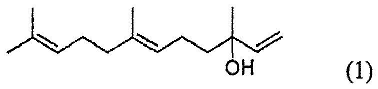 with an oxidizing agent in the presence of a vanadium complex of the following general formula (2): 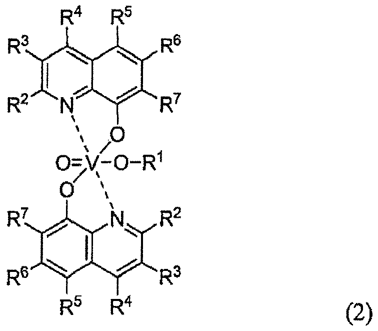 wherein
R¹ represents an alkyl group having 1 to 20 carbon atoms, a cycloalkyl group having 3 to 8 carbon atoms, an alkenyl group having 2 to 20 carbon atoms, an aryl group having 6 to 18 carbon atoms, an aralkyl group having 7 to 20 carbon atoms, or an acyl group having 2 to 7 carbon atoms, and
R², R³, R⁴, R⁵, R⁶ and R⁷ each independently represent a hydrogen atom, alkyl group having 1 to 6 carbon atoms, haloalkyl group having I to 6 carbon atoms, alkoxy group having 1 to 6 carbon atoms, alkylthio group having 1 to 6 carbon atoms, phenyl group, nitro group, cyano group, carboxyl group or halogen atom.

2. The production method according to claim 1, wherein the vanadium complex of the general formula (2) is prepared by reacting a vanadium compound selected from a compound of the following general formula (4a): 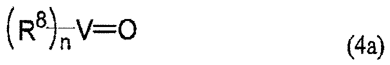 wherein
n represents 2 or 3, and R⁸ represents an R'O- group or either alone or together represents an alkanedionate having 2 to 8 carbon atoms, where R' represents an alkyl group having 1 to 6 carbon atoms or a cycloalkyl group having 3 to 6 carbon atoms,
or a metavanadate salt with an 8-quinolinol derivative of the following general formula (6): 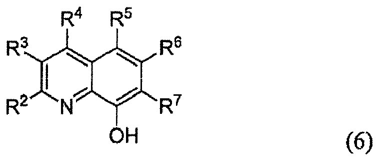 wherein
R², R³, R⁴, R⁵, R⁶ and R⁷ are the same as defined in claim 1,
and an oxidizing agent as necessary in the presence (case in which R¹ ≠ R') or absence (cases in which R¹ = R') of a compound of the following general formula (5):
R¹OH (5)
wherein
R¹ is the same as defined in claim 1.

3. The production method according to claim 1 or 2, wherein the vanadium complex of the general formula (2) is formed in the reaction system.

4. A method for producing farnesal of the following formula (3): 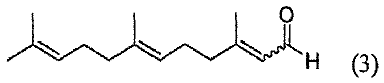 comprising: reacting (E)-nerolidol of the following formula (1): 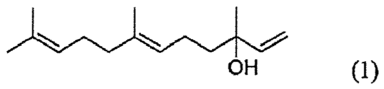 with an oxidizing agent in the presence of a vanadium compound selected from a compound of the following general formula (4a): 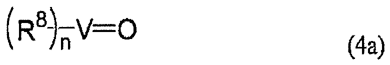 wherein
n and R⁸ are the same as defined in claim 1,
or a metavanadate salt, a compound of the following general formula (5):
R¹OH (5)
wherein
R¹ is the same as defined in claim 1,
and an 8-quinolinol derivative of the following general formula (6): 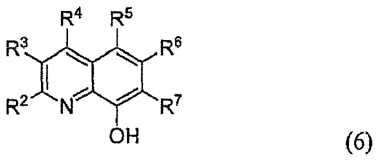 wherein
R², R³, R⁴, R⁵, R⁶ and R⁷ are the same as defined in claim 1.

5. The production method according to any of claims 1 to 4, wherein R¹ is an alkyl group having 1 to 20 carbon atoms.

6. The production method according to any of claims 1 to 4, wherein R¹ is a 3,7,11-trimethyl-1,6,10-dodecatrien-3-yl group or 3,7,11-trimethyl-2,6,10-dodecatrienyl group.

7. The production method according to any of claims 2 to 6, wherein the vanadium compound selected from a compound of the general formula (4a) or a metavanadate salt is triisopropoxyvanadium (V) oxide, bis(acetylacetonato)oxovanadium (IV) or ammonium metavanadate.

8. The production method according to any of claims 1 to 3, wherein the amount of the vanadium complex of the general formula (2) used is 0.05 moles to 0.2 moles based on 1 mole of the (E)-nerolidol of formula (1).

9. The production method according to any of claims 1 to 8, wherein the oxidizing agent is air, oxygen, dimethylsulfoxide or tetramethylenesulfoxide.

10. The production method according to any of claims 1 to 9 carried out in an aromatic solvent.

11. The production method according to any of claims 1 to 10 carried out at a temperature of 80°C to 140°C.

12. A vanadium complex of the following general formula (2a): 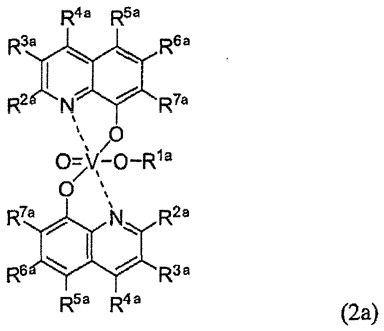 wherein
R^{1a} represents an alkyl group having 1 to 20 carbon atoms, an alkenyl group having 2 to 20 carbon atoms, an aryl group having 6 to 18 carbon atoms or an aralkyl group having 7 to 20 carbon atoms, and
R^{2a}, R^{3a}, R^{4a}, R^{5a}, R^{6a} and R^{7a} each independently represent a hydrogen atom, alkyl group having 1 to 6 carbon atoms, haloalkyl group having 1 to 6 carbon atoms, alkoxy group having 1 to 6 carbon atoms, phenyl group, nitro group, cyano group, carboxyl group or halogen atom,
provided that, in the case R^{2a}, R^{3a}, R^{4a}, R^{5a}, R^{6a} and R^{7a} are all hydrogen atoms, R^{1a} is not an alkyl group having 1 to 12 carbon atoms, an allyl group or a benzyl group, in the case R^{2a} is a methyl group and R^{3a}, R^{4a}, R^{5a}, R^{6a} and R^{7a} are hydrogen atoms, R^{1a} is not an alkyl group having 1 to 4 carbon atoms, in the case R^{2a}, R^{3a}, R^{4a}, R^{6a} and R^{7a} are hydrogen atoms and R^{5a} is a methyl group or a nitro group, R^{1a} is not an ethyl group, and in the case R^{2a}, R^{3a}, R^{4a}, R^{6a} and R^{7a} are hydrogen atoms and R^{5a} is a halogen atom or R^{2a}, R^{3a}, R^{4a} and R^{6a} are hydrogen atoms and R^{5a} and R^{7a} are halogen atoms, R^{1a} is not an alkyl group having 1 to 5 carbon atoms.

13. A method for producing a vanadium complex of the following general formula (2a): 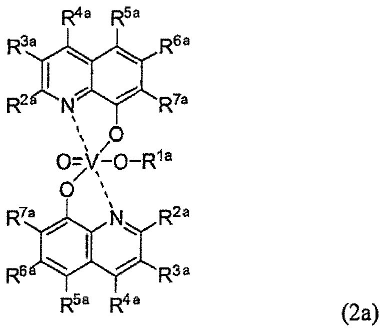 wherein
R^{1a}, R^{2a}, R^{3a}, R^{4a}, R^{5a}, R^{6a} and R^{7a} are the same as defined in claim 12,
comprising: reacting a vanadium compound selected from a compound of the following general formula (4a): 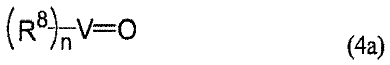 wherein
n and R⁸ are the same as defined in claim 2,
or a metavanadate salt with an 8-quinolinol derivative of the following general formula (6a): 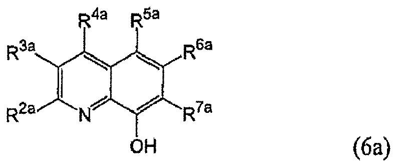 wherein
R^{2a}, R^{3a}, R^{4a}, R^{5a}, R^{6a} and R^{7a} are the same as defined in claim 12,
and an oxidizing agent as necessary, in the presence (in the case R^{a1} ≠ R') or in the absence (in the case R^{1a} = R') of an alcohol of the following general formula (5a):
R^{1a}OH (5a)
wherein
R^{1a} is the same as defined in claim 12.

## Amended claims

### Amended claims under Art. 19.1 PCT

1. A method for producing farnesal of the following formula (3): 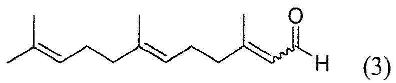 comprising: reacting (E)-nerolidol of the following formula (1): 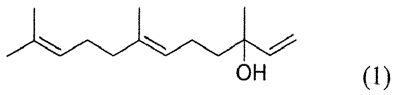 with an oxidizing agent in the presence of a vanadium complex of the following general formula (2): 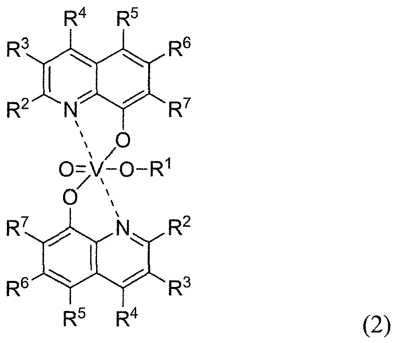 wherein
R¹ represents an alkyl group having 1 to 20 carbon atoms, a cycloalkyl group having 3 to 8 carbon atoms, an alkenyl group having 2 to 20 carbon atoms, an aryl group having 6 to 18 carbon atoms, an aralkyl group having 7 to 20 carbon atoms, or an acyl group having 2 to 7 carbon atoms, and
R², R³, R⁴, R⁵, R⁶ and R⁷ each independently represent a hydrogen atom, alkyl group having 1 to 6 carbon atoms, haloalkyl group having 1 to 6 carbon atoms, alkoxy group having 1 to 6 carbon atoms, alkylthio group having 1 to 6 carbon atoms, phenyl group, nitro group, cyano group, carboxyl group or halogen atom.

2. The production method according to claim 1, wherein the vanadium complex of the general formula (2) is prepared by reacting a vanadium compound selected from a compound of the following general formula (4a): 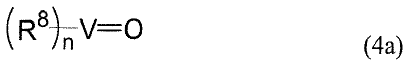 wherein
n represents 2 or 3, and R⁸ represents an R'O- group or either alone or together represents an alkanedionate having 2 to 8 carbon atoms, where R' represents an alkyl group having 1 to 6 carbon atoms or a cycloalkyl group having 3 to 6 carbon atoms,
or a metavanadate salt with an 8-quinolinol derivative of the following general formula (6): 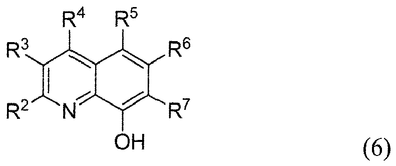 wherein
R², R³, R⁴, R⁵, R⁶ and R⁷ are the same as defined in claim 1,
and an oxidizing agent as necessary in the presence (case in which R¹≠ R') or absence (cases in which R¹ = R') of a compound of the following general formula (5):
R¹OH (5)
wherein
R¹ is the same as defined in claim 1.

3. The production method according to claim 1 or 2, wherein the vanadium complex of the general formula (2) is formed in the reaction system.

4. A method for producing farnesal of the following formula (3): 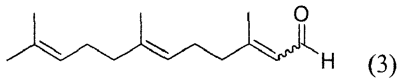 comprising: reacting (E)-nerolidol of the following formula (1): 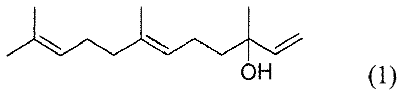 with an oxidizing agent in the presence of a vanadium compound selected from a compound of the following general formula (4a): 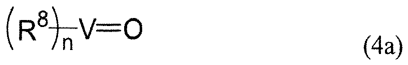 wherein
n and R⁸ are the same as defined in claim 1,
or a metavanadate salt, a compound of the following general formula (5):
R¹OH (5)
wherein
R¹ is the same as defined in claim 1,
and an 8-quinolinol derivative of the following general formula (6): 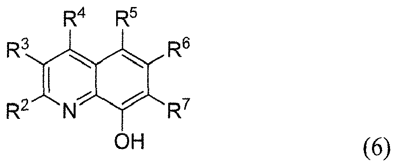 wherein
R², R³, R⁴, R⁵, R⁶ and R⁷ are the same as defined in claim 1.

5. The production method according to any of claims 1 to 4, wherein R¹ is an alkyl group having 1 to 20 carbon atoms.

6. The production method according to any of claims 1 to 4, wherein R¹ is a 3,7,11-trimethyl-1,6,10-dodecatrien-3-yl group or 3,7,11-trimethyl-2,6,10-dodecatrienyl group.

7. The production method according to any of claims 2 to 6, wherein the vanadium compound selected from a compound of the general formula (4a) or a metavanadate salt is triisopropoxyvanadium (V) oxide, bis(acetylacetonato)oxovanadium (IV) or ammonium metavanadate.

8. The production method according to any of claims 1 to 3, wherein the amount of the vanadium complex of the general formula (2) used is 0.05 moles to 0.2 moles based on 1 mole of the (E)-nerolidol of formula (1).

9. The production method according to any of claims 1 to 8, wherein the oxidizing agent is air, oxygen, dimethylsulfoxide or tetramethylenesulfoxide.

10. The production method according to any of claims 1 to 9 carried out in an aromatic solvent.

11. The production method according to any of claims 1 to 10 carried out at a temperature of 80°C to 140°C.

12. (amended) A vanadium complex of the following general formula (2a): 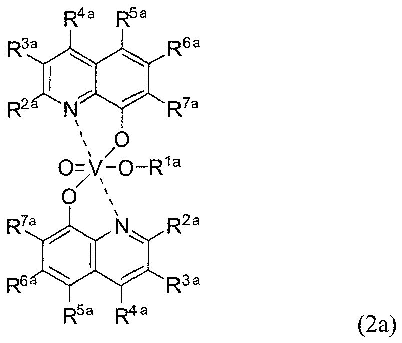 wherein
R^{1a} represents an alkyl group having 1 to 20 carbon atoms, an alkenyl group having 2 to 20 carbon atoms, an aryl group having 6 to 18 carbon atoms or an aralkyl group having 7 to 20 carbon atoms, and
R^{2a}, R^{3a}, R^{4a}, R^{5a}, R^{6a} and R^{7a} each independently represent a hydrogen atom, alkyl group having 1 to 6 carbon atoms, haloalkyl group having 1 to 6 carbon atoms, alkoxy group having 1 to 6 carbon atoms, phenyl group, nitro group, cyano group, carboxyl group or halogen atom,
provided that, in the case R^{2a}, R^{3a}, R^{4a}, R^{5a}, R^{6a} and R^{7a} are all hydrogen atoms, R^{1a} is not an alkyl group having 1 to 12 carbon atoms, an allyl group, a benzyl group or a 4-methoxybenzyl group, in the case R^{2a} is a methyl group and R^{3a}, R^{4a}, R^{5a}, R^{6a} and R^{7a} are hydrogen atoms, R^{1a} is not an alkyl group having 1 to 4 carbon atoms, in the case R^{2a}, R^{3a}, R^{4a}, R^{6a} and R^{7a} are hydrogen atoms and R^{5a} is a methyl group or a nitro group, R^{1a} is not an ethyl group, and in the case R^{2a}, R^{3a}, R^{4a}, R^{6a} and R^{7a} are hydrogen atoms and R^{5a} is a halogen atom or R^{2a}, R^{3a}, R^{4a} and R^{6a} are hydrogen atoms and R^{6a} and R^{7a} are halogen atoms, R^{1a} is not an alkyl group having 1 to 5 carbon atoms.

13. A method for producing a vanadium complex of the following general formula (2a): 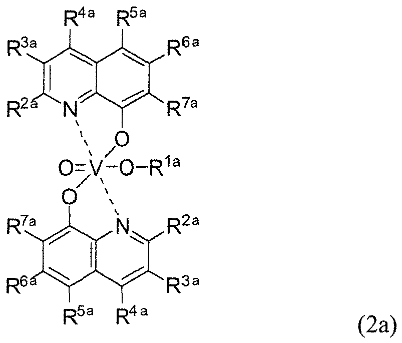 wherein
R^{1a}, R^{2a}, R^{3a}, R^{4a}, R^{5a}, R^{6a} and R^{7a} are the same as defined in claim 12,
comprising: reacting a vanadium compound selected from a compound of the following general formula (4a): 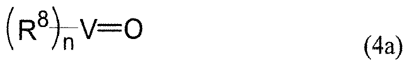 wherein
n and R⁸ are the same as defined in claim 2,
or a metavanadate salt with an 8-quinolinol derivative of the following general formula (6a): 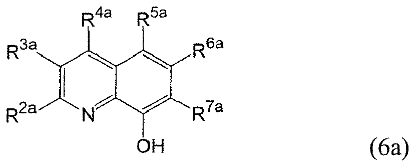 wherein
R^{2a}, R^{3a}, R^{4a}, R^{5a}, R^{6a} and R^{7a} are the same as defined in claim 12,
and an oxidizing agent as necessary, in the presence (in the case R^{1a} ≠ R') or in the absence (in the case R^{1a} = R') of an alcohol of the following general formula (5a):
R^{1a}OH (5a)
wherein
R^{1a} is the same as defined in claim 12.
